Europäisches Patentamt

European Patent Office

Office européen des brevets

(11) Publication number: **0 358 813**
**A1**

## EUROPEAN PATENT APPLICATION

(21) Application number: 88115228.4

(51) Int. Cl.5: **A61F 13/15**

(22) Date of filing: **16.09.88**

(43) Date of publication of application:
**21.03.90 Bulletin 90/12**

(84) Designated Contracting States:
**DE ES FR GB IT SE**

(71) Applicant: **Toyo Eizai Kabushiki Kaisha**
**No. 45-2, Handa-otsu Kanada-machi**
**Kawanoe-shi Ehime-ken(JP)**

(72) Inventor: **Masashi, Uda**
**19-23, Gein 3-chome**
**Minoo-shi Osaka-fu(JP)**

(74) Representative: **Thomsen, Dieter, Dr.rer.nat.**
**Kaiser-Ludwig-Platz 6**
**D-8000 München 2(DE)**

(54) **A disposable diaper.**

(57) A disposable diaper having three layers of a permeable sheet (1), an absorbent sheet (3) and a sealing sheet (2), wherein the sealing sheet (2) has a fold tucked along the length of the sealing sheet (2) so that the permeable sheet (1) and the absorbent sheet (3) respectively have folds founded by the fold of the sealing sheet (2).

# Fig. 1

EP 0 358 813 A1

## A disposable diaper

The present invention relates to a disposable diaper used for babies and bedridden patients (hereinafter referred to as "wearer"), and more particularly, to a disposable diaper capable of providing a liquidtight fit to the wearer's skin, thereby avoiding the leakage of body secretion including urine and excretion.

Nowadays disposable diapers are becoming more and more popular because they are sanitary and save the laundering toil after each use. In order to explain the background of the present invention, the known disposable diapers will be described by reference to Figure 2:

A disposable diaper (hereinafter referred to as "diaper") has three layers of a permeable nonwoven sheet 1, an absorbent sheet 3 and a sealing sheet 2 (e.g. a plastic film), the absorbent sheet 3 having a smaller area than the sheets 1 and 2. The sheets 1 and 2 are thermally jointed to each other at their peripheries, with the absorbent sheet interlocated therebetween. The sealing sheet 2 is provided with two elastic strings ($E_1$) in its opposite side margins, the elastic strings being stitched in a stretched state along the length (L) of the sealing sheet 2. When necessary, a further elastic string ($E_2$) can be provided in each margin along the width (S) of the sealing sheet 2.

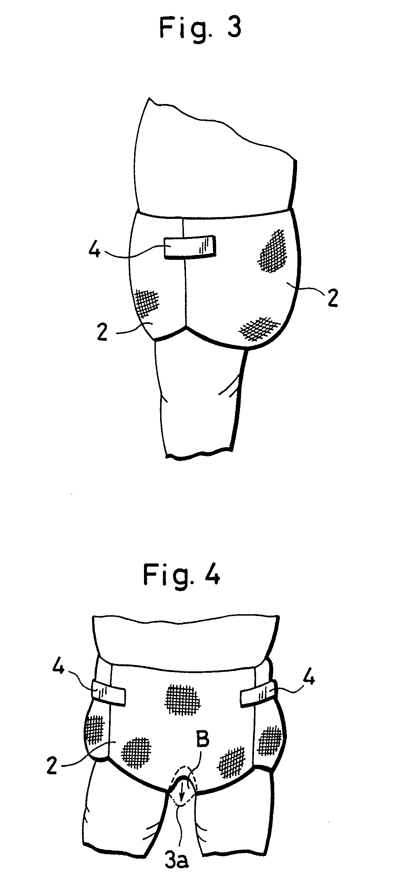

Fig. 3 shows the diaper in use. The wearer wears the diaper in such a manner that a narrow portion (N) of the permeable sheet 1 covers the crotch of the wearer, and wider portions (W) thereof cover the belly and the back. The abutting edges of the sealing sheet 2 are fastened to each other by fastener tapes 4 so that the diaper securely wraps up the wearer's waist.

The absorbent sheet 3, which keeps direct contact with the wearer's skin, absorbs urine and excretion. The problem is that as the wearer moves, the absorbent sheet gradually dislocates from the crotch, and enlarges the gap between the skin and the absorbent sheet, thereby causing a leakage of urine and excretion through the gap. Figure 4 shows a typical example in which a part 3a of the absorbent sheet becomes loose, enlarging a gap (B) in the crotch. One of the reasons for enlarging the gap (a) is that this part of diaper is previously made wider than an average width of babies' or adults' crotches, depending upon the use for babies or for adults. As the part 3a becomes impregnatd with urine or excretion, it tends to hung heavily downward. Another reason for enlarging the gap (B) is that this part 3a can not have a sufficient pressure by the elastic strings ($E_1$), and if any, ($E_2$) in the sealing sheet 2. Whatever the reason may be, a leakage due to the loose fit to the skin is a fatal defect for diapers.

The present invention aims at overcoming the problems encountered by the known disposable diapers, and has for its object to provide a diaper securing a constant watertight fit to the skin, thereby avoiding a possible leakage of body secretion including urine and excretion.

Another object of the present invention is to provide a disposable diaper securing a pleasant touch to the wearer's skin.

Other objects and advantages of the present invention will become more apparent from the following detailed description, when taken in conjunction with the accompanying drawings which show, for the purpose of illustration only, one embodiment in accordance with the present invention.

According to the present invention there is provided a disposable diaper having three layers of a permeable sheet, an absorbent sheet and a sealing sheet, wherein the sealing sheet has a fold tucked along the length of the sealing sheet so that the permeable sheet and the absorbent sheet respectively have folds founded by the fold of the sealing sheet.

### BRIEF DESCRIPTION OF THE DRAWINGS

Figure 1 is a cross-sectional view showing a diaper according to the present invention;

Figure 2 is a perspective view showing a known disposable diaper;

Figure 3 is a side view showing a disposable diaper worn by a wearer;

Figure 4 is a front view showing a loose crotch portion of the known diaper;

Figures 5, 6 and 7 are cross-sectional view showing various modifications of the present invention; and

Figure 8 is a bottom view showing the diaper according to the present invention.

### DETAILED DESCRIPTION OF THE INVENTION

A diaper according to the present invention basically has the same structure as that of the known diaper shown in Figure 2, but is different from it in the following manner:

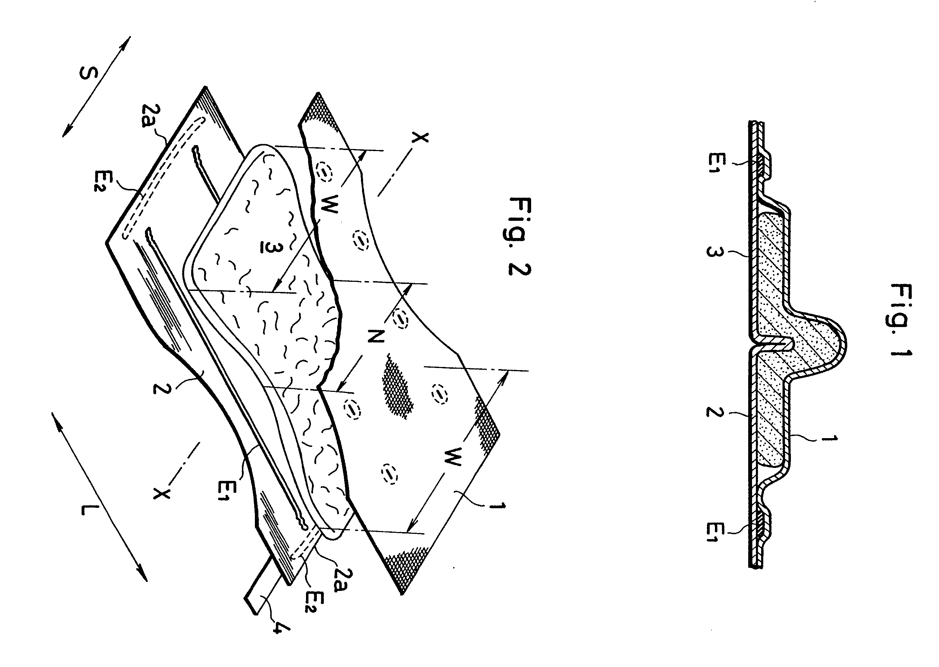

As shown in Figure 1, the lowermost sealing sheet 2 has a fold tucked along the length thereof. The inner sides of fold are bonded so as not to open. The permeable sheet 1 and the absorbent sheet 3 overlaid on the sealing sheet 2 are naturally projected by the fold of the sealing sheet 2, thereby forming the respective folds. Since the fundation fold of the sealing sheet 2 is rigid, the folds of the permeable sheet 1 and the absorbent sheet 3 are prevented from losing the shapes even when they are pressed against the wearer's skin.

When a wearer wears the diaper, the folds of the permeable sheet 1 and the absorbent sheet 3 come into abutment with the wearer's crotch, and fill in the gap (B) between the diaper and the skin.

The size, height and length of the folds of the three sheets 1, 2 and 3 depend on the wearer's physical dimension and sex. In the market the purchaser chooses an appropriate size for the wearer.

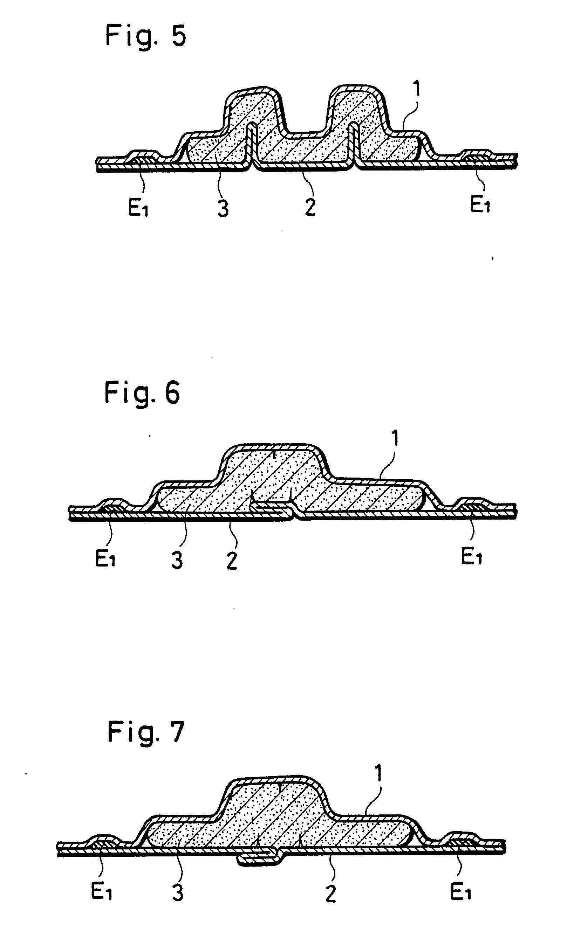

Figure 5 shows a modified version which has a couple of folds, and Figure 6 shows a further modified version which is obtained by flattening the version of Figure 5 to form one relatively flat fold. Figure 7 shows a version similar in appearance to the version of Figure 6, but is different in that the sealing sheet 2 is folded outwards.

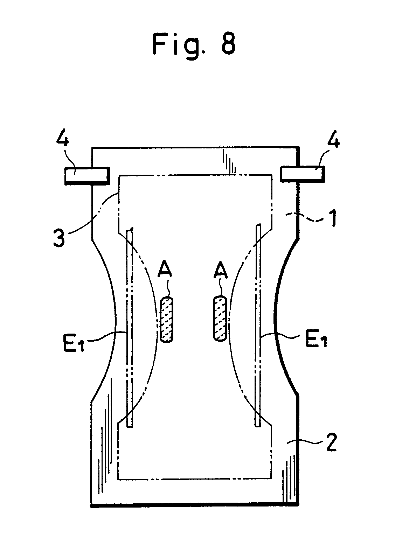

Referring to Figure 8, a method of forming the folds will be described:

The sealing sheet 2 is provided with adhesive-coated zones (A), the adhesive being preferably of a kind which can mutually adheres but can not adhere to an object which has no coating of the same adhesive. For such adhesives acrylic-base adhesive, EVA emulsion type adhesive, and a latex-base adhesive can be used. Other fastening methods can be applied; for example, a combination of a releasable tape and a thermally melting adhesive such as SBS-base, olefine-base, SIS-base, and EVA-base adhesive can also be used. Any other known methods can be selectively used.

The permeable sheets 1 and the sealing sheet 2 are thermally jointed to each other at their peripheries, with the absorbent sheet 3 being interlocated therebetween. The sealing sheet 2 is provided with two elastic strings ($E_1$) in its opposite side margins, the elastic strings being in a state stretched in the length (L) of the sealing sheet 2. Optionally a further elastic string ($E_2$) is provided in each margin along the width (S) of the sealing sheet 2. Finally fasteners 4 are symmetrically provided at opposite side of the sealing sheet 2 whereby the diaper securely wraps up the wearer's waist when it is worn.

## Claims

1. A disposable diaper having three layers of a permeable sheet, an absorbent sheet and a sealing sheet, wherein the sealing sheet has a fold tucked in the direction of length so that the permeable sheet and the absorbent sheet respectively have folds founded by the fold of the sealing sheet.

2. A disposable diaper having three layers of a permeable sheet, an absorbent sheet and a sealing sheet, wherein the sealing sheet has adhesive-coated zones on the bottom surface, the adhesive-coated zones being located symmetrically of the lengthwise axis of the sealing sheet so that when they are met, the two zones adhere to each other to form a firm fold on the top surface of the sealing sheet.

3. A disposable diaper having three layers of a permeable sheet, an absorbent sheet and a sealing sheet, wherein the sealing sheet has fastener tapes on its bottom surface, the fastener tapes being provided symmetrically of the lengthwise axis of the sealing sheet so that when they are met, the two tapes adhere to each other to form a firm fold on the top surface of the sealing sheet.

4. A disposable diaper as set forth in any of the claims 1 to 3, wherein the sealing sheet has a single fold.

5. A disposable diaper as set forth in any of the claims 1 to 3, wherein the the sealing sheet has two folds tucked in parallel.

# Fig. 1

# Fig. 2

# Fig. 3

# Fig. 4

Fig. 5

Fig. 6

Fig. 7

# Fig. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-2331355 (H. L. STRONGSON)<br>* page 2, column 2, line 12 - line 28; claim 3; figures 8-12 * | 1 | A61F13/15 |
| A | | 2, 4 | |
| Y | US-A-3968799 (KIMBERLY-CLARK CORPORATION)<br>* column 2, line 28 - line 67; figures 1-6 * | 1 | |
| A | | 3, 5 | |
| A | GB-A-1171496 (JOHNSON & JOHNSON)<br>* page 2, line 85 - page 3, line 27; figures 1-5 * | 1 | |
| A | US-A-3530859 (L. HEIMOWITZ)<br>* column 3, line 4 - line 68; figures 1-3 * | 1, 5 | |
| A | US-A-2571577 (R. HOWARD)<br>* column 3, line 8 - line 34; figures 6, 7 * | 1, 4 | |
| A | US-A-4041950 (J. L. JONES SR) | | |
| A | US-A-3995640 (COLGATE-PALMOLIVE COMPANY) | | TECHNICAL FIELDS SEARCHED (Int. Cl.5 )<br><br>A41B<br>A61F |

The present search report has been drawn up for all claims

| Place of search | Date of completion of the search | Examiner |
|---|---|---|
| THE HAGUE | 01 JUNE 1989 | GARNIER F.M.A.C. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                   
& : member of the same patent family, corresponding
document